# EUROPEAN PATENT APPLICATION

(11) **EP 2 845 580 A1**
(43) Date of publication of application: **11.03.2015**
(21) Application number: 13781614.6
(22) Date of filing: 26.04.2013
(51) Int. Cl.: A61K 8/19, A61K 8/36, A61K 8/97, A61Q 19/10

(54) **COMPOSITION FOR BATH AGENT**

(30) Priority: 27.04.2012 JP 2012103534
(71) Applicant: Kao Corporation, Chuo-Ku Tokyo 103-8210 (JP)
(72) Inventor: ASHIDA, Arisa, Tokyo 131-8501 (JP); HORI, Takaaki, Tokyo 131-8501 (JP); YAMAKI, Kazuhiro, Tokyo 131-8501 (JP); NOMURA, Tomoko, Haga-gun Tochigi 321-3497 (JP); SUGATA, Keiichi, Haga-gun Tochigi 321-3497 (JP)
(74) Representative: Hoffmann Eitle
(86) International application number: PCT/JP2013/062440
(87) International publication number: WO 2013/162013

(57) **Abstract**

Provided is a bath agent composition capable of satisfactorily exhibiting blood circulation promoting effect by generating carbon dioxide gas by comprising a carbonate and an organic acid therein and capable of satisfactorily exhibiting an additional effect by imparting high preservation stability. The bath agent composition comprises the following components: (A) a carbonate comprising 30 mass% or more of a dialkali metal carbonate (A1); (B) an organic acid; and (C) a CGRP response promoter.

## Description

### Field of the Invention

The present invention relates to a bath agent composition.

### Background of the Invention

Heretofore, a bath agent obtained by blending a carbonate and an organic acid has been widely known as an excellent bath agent because the agent generates bubbles of carbon dioxide gas in bath water, and the carbon dioxide gas provides a blood circulation promoting effect. Further, a study for providing an additional effect or a synergistic effect by further blending various components in the bath agent has been made.

For example, a crude drug such as ginseng has been known as a component capable of being blended in a so-called foaming bath agent obtained by blending a carbonate and an organic acid (see Patent Documents 1 and 2), and a foaming bath agent that includes such component and thus can synergistically enhance a blood circulation promoting effect and moist feeling of skin after bathing has been developed (see Patent Document 3). In addition, a bath agent having a throbbing pain alleviating effect provided by using an extract obtained from a plant of the Cupressaceae family has also been developed (Patent Document 4).

### Citation List

### Patent Document

[Patent Document 1] JP-A-62-234015
[Patent Document 2] JP-B-7-546
[Patent Document 3] JP-A-60-215618
[Patent Document 4] JP-A-11-158058

### Summary of the Invention

The present invention provides a bath agent composition, comprising the following components (A) to (C):
(A) a carbonate comprising 30 mass% or more of a dialkali metal carbonate (A1);
(B) an organic acid; and
(C) a CGRP response promoter.

### Detailed Description of the Invention

In a conventional bath agent obtained by blending a carbonate and an organic acid, only blending the components described above may deteriorate preservation stability due to the generation of carbon dioxide gas and may not satisfactorily provide an additional effect or a synergistic effect which is expected.

Therefore, the present invention provides a bath agent composition capable of satisfactorily exhibiting a blood circulation promoting effect due to the generation of carbon dioxide gas, wherein the composition comprises a carbonate and an organic acid and is capable of satisfactorily exhibiting an additional effect by imparting high preservation stability.

Further, the inventors of the present invention made various studies, and found that, when a carbonate comprising a specific amount of a dialkali metal carbonate and an organic acid were combined with a CGRP response promoter, good dispersibility of the composition was maintained to provide excellent preservation stability, and the inventors surprisingly found that continuous use of the composition enhanced vascular responsiveness to heat load. Thus, the present invention was completed.

The bath agent composition of the present invention is possible not only to favorably generate carbon dioxide gas to enhance the blood circulation promoting effect but also to satisfactorily enhance the CGRP response promoter's effect over time because of the high preservation stability provided even when the composition further contains the CGRP response promoter. In particular, when the composition is continuously used, the composition can provide excellent vascular responsiveness to heat load.

### Brief Description of the Drawings

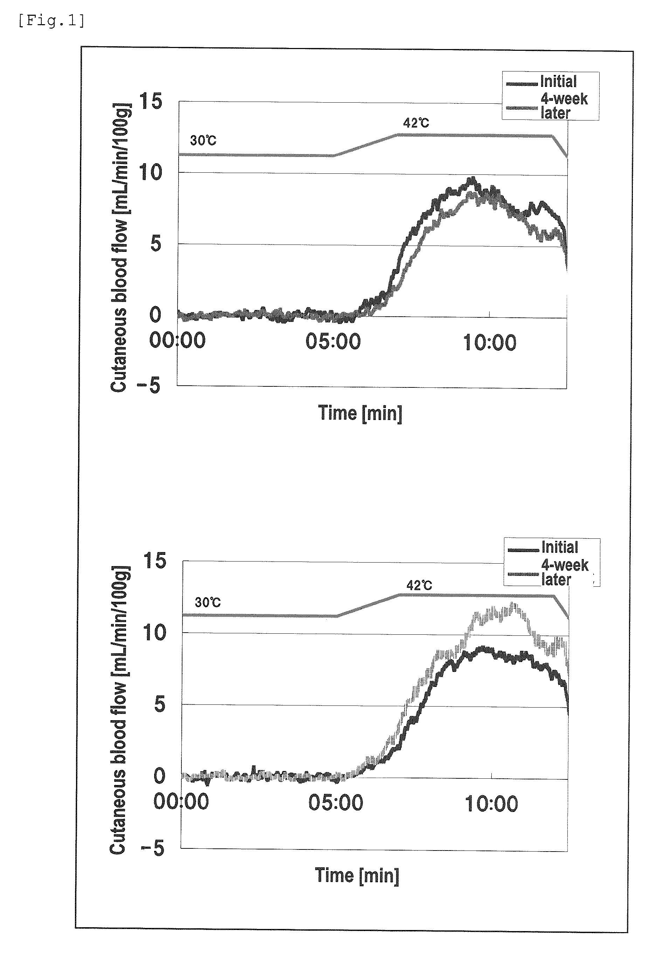

FIG. 1 are graphs each showing a comparison of changes in the cutaneous blood flow between the day before the start of 4-week continuous bathing (foot bathing) (initial) and the day after the continuous use (4 weeks later). The vertical axis represents cutaneous blood flow (mL/min/100 g), and the horizontal axis represents time (min). FIG. 1(a) shows the case of normal hot water (Comparative Example 5), and FIG. 1(b) shows the case of using the bath agent composition of the present invention (Example 1). Both graphs show observation results of blood flow where initial skin temperature was adjusted to 30°C, the skin was acclimated well and subjected to heat load, the skin temperature was increased to 42°C, kept at 42°C for 5 minutes and returned to 30°C again. It should be noted that the cutaneous blood flow was measured in the same manner as the method for evaluation of vascular responsiveness improving effect to heat load in Test Example 3.

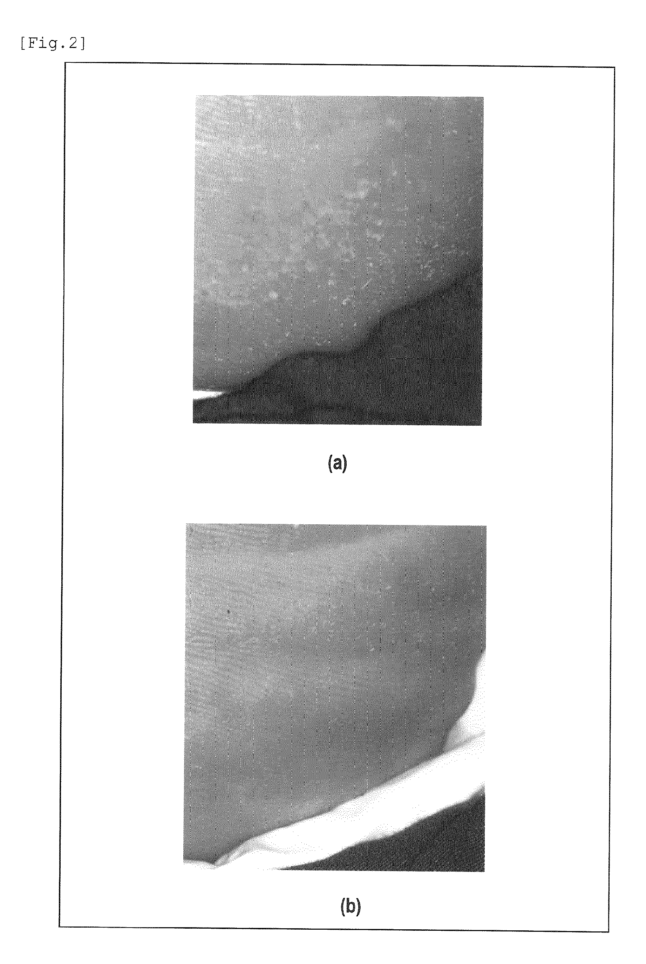

FIG. 2 are photographs each showing the horny layer of the heel in the case of 4-week continuous bathing (foot bathing) using the bath agent composition of the present invention (Example 1). FIG. 2(a) shows the heel before bathing (foot bathing), and FIG. 2 (b) shows the heel after 4-week continuous bathing (foot bathing) .

Hereinafter, the present invention is described in detail.

The bath agent composition of the present invention comprises a carbonate (A) including 30 mass% or more of a dialkali metal carbonate (A1). When the bath agent composition of the present invention is put in bath water, the carbonate (A) can generate carbon dioxide gas together with an organic acid (B) to be described later to provide a blood circulation promoting effect, resulting in imparting good warm feeling to the skin. The component (A) comprises 30 mass% or more of the dialkali metal carbonate (A1) in the component (A). That is, 30 mass% or more of the dialkali metal carbonate (A1) is comprised in the total amount of the component (A). This can maintain high preservation stability, can ensure a satisfactory amount of carbon dioxide gas generated to provide an excellent blood circulation promoting effect, and can provide a satisfactory effect provided by a CGRP response promoter (C) to be described later. Examples of the dialkali metal carbonate (A1) include dialkali metal carbonates such as sodium carbonate and potassium carbonate, and from the viewpoint of blood circulation promoting effect, sodium carbonate is preferred. The amount of the dialkali metal carbonate (A1) in the component (A) is 30 mass% or more, preferably from 40 to 100 mass%, more preferably from 50 to 100 mass%, even more preferably from 60 to 100 mass%, from the viewpoint of ensuring the amount of carbon dioxide gas generated and the viewpoint of maintaining good preservation stability.

The bath agent composition of the present invention may include a carbonate (A2) excluding the dialkali metal carbonate (A1) in the component (A). Examples of the component (A2) include: a monoalkali metal carbonate such as sodium hydrogen carbonate or potassium hydrogen carbonate; and a carbonate of a divalent or higher-valent metal such as calcium carbonate. One kind may be used alone, or two or more kinds may be used in combination. Of those, from the viewpoint of ensuring high preservation stability, a monoalkali metal carbonate is preferred, and sodium hydrogen carbonate or potassium hydrogen carbonate is more preferred. From the viewpoint of ensuring high preservation stability, the amount of the monoalkali metal carbonate in 100 mass% of the component (A) is preferably 60 mass% or less, more preferably 50 mass% or less, even more preferably from 0 to 40 mass%. Further, the component (A) preferably includes no monoalkali metal carbonate except when monoalkali metal carbonate is inevitably contained within another component.

The amount of the carbonate as the component (A) in the bath agent composition of the present invention is preferably from 20 to 70 mass%, more preferably from 25 to 65 mass%, even more preferably from 30 to 60 mass%, from the viewpoint of ensuring an amount of carbon dioxide gas generated to provide a satisfactory blood circulation promoting effect.

The bath agent composition of the present invention comprises an organic acid (B). The component (B) is preferably an organic acid that is solid at room temperature (25°C) such as malic acid, tartaric acid, citric acid, maleic acid, succinic acid, phthalic acid, fumaric acid, glutaric acid, adipic acid, benzoic acid, salicylic acid and oxalic acid. One kind may be used alone, or two or more kinds may be used in combination. Of those, one or more kinds selected from citric acid, fumaric acid, malic acid, tartaric acid, succinic acid and oxalic acid are more preferred, and citric acid or fumaric acid is even more preferred.

The amount of the component (B) in the bath agent composition of the present invention is preferably from 20 to 70 mass%, more preferably from 25 to 65 mass%, even more preferably from 30 to 60 mass%, from the viewpoint of ensuring an amount of carbon dioxide gas generated to provide a satisfactory blood circulation promoting effect.

The bath agent composition of the present invention comprises a CGRP response promoter (C). The component (C) is an agent capable of promoting CGRP responsiveness of a cell. CGRP responsiveness is CGRP receptor expression or any kind of activity that takes place in a cell that has a CGRP receptor. Specifically, for example, when the CGRP is bound to a CGRP receptor of a cell, intracellular cAMP increases through activation of adenylate cyclase via G-protein. As a result, for example, in a vascular smooth muscle cell, K⁺ channel is opened via activation of protein kinase A. Alternatively, in a vascular endothelial cell, eNOS is activated via activation of protein kinase A to promote production of NO. Further, the NO produced acts on the vascular smooth muscle cell to open the K⁺ channel through activation of intracellular cGMP via NO.

Heretofore, carbon dioxide gas-generating substances such as the carbonate (A) and the organic acid (B) have been used for many bath agent compositions as components capable of generating carbon dioxide gas that provides an excellent blood circulation promoting effect. On the other hand, the bath agent composition of the present invention comprises both the carbon dioxide gas-generating substances and the CGRP response promoter (C). Therefore, the composition can relax the vascular smooth muscle and, together with carbon dioxide gas, can promote dilation of the blood vessel to provide an excellent blood circulation promoting effect and to improve skin circulation or skin metabolism, resulting in enhancing vascular responsiveness. Specifically, for example, when the bath agent composition of the present invention is used for bathing, the vascular responsiveness to heat load can be enhanced. Further, for skin having a decreased water amount in the horny layer to cause progressive hardening, the composition can suppress an additional decrease in the water amount to provide soft skin. Further, when the composition contains another component such as an oily component or the like to be described later, the composition can promote absorption of the component to the skin.

Examples of the component (C) include ginseng, hiba arborvitae, cucumber, butcher's broom, Amur cork tree, *Hemerocallis fulva,* rose balsam, bladderwrack, birch, rice protein, rice oil, *Aloe vera,* moutan bark, laminaria, star fruit, cornflower, lemongrass, apricot kernel, lemon, sweet marjoram, horse chestnut, royal jelly, rose hip, and extracts thereof. One kind may be used alone, or two or more kinds may be used in combination.

The "ginseng" refers to *Panax ginseng* belonging to the genus *Panax* of the Araliaceae family, and the "ginseng extract" refers to an extract obtained from the ginseng. The ginseng extract may be an extract obtained from any part of the ginseng, for example, the whole plant, leaf, stem, germ, flower, bud, xylem, bark, thallus, root, rhizome, pseudobulb, corm, tuber, seed, fruit, sclerotium or resin, or an extract obtained from a combination thereof. Of those, an extract from the root is preferred.

The "hiba arborvitae" refers to *Thujopsis dolabrata* belonging to the genus *Thujopsis* of the Cupressaceae family, and the "hiba arborvitae extract" refers to an extract obtained from the hiba arborvitae. The hiba arborvitae extract may be an extract obtained from any part of the hiba arborvitae, for example, the whole plant, leaf, stem, germ, flower, bud, xylem, bark, thallus, root, rhizome, pseudobulb, corm, tuber, seed, fruit, sclerotium or resin, or an extract obtained from a combination thereof. Of those, an extract from the leaf or branch is preferred.

The "cucumber" refers to *Cucumis sativus* belonging to the genus *Cucumis* of the Cucurbitaceae family, and the "cucumber extract" refers to an extract obtained from the cucumber. The cucumber extract may be an extract obtained from any part of the cucumber, for example, the whole plant, leaf, stem, germ, flower, bud, xylem, bark, thallus, root, rhizome, pseudobulb, corm, tuber, seed, fruit, sclerotium or resin, or an extract obtained from a combination thereof. Of those, an extract from the fruit is preferred.

Further, the "butcher's broom" refers to *Ruscus aculeatus* of the Liliaceae family, the "Amur cork tree" refers to *Phellodendron amurense* of the Rutaceae family, the *"Hemerocallis fulva"* refers to *Hemerocallis fulva* of the Liliaceae family, the "rose balsam" refers to *Impatiens balsamina* of the Balsaminaceae family, the "bladderwrack" refers to *Fucus vesiculosus* of the Fucaceae family, the "birch" refers to a plant belonging to the genus *Betula* of the Betulaceae family, preferably *Betula pendula,* the *"Aloe vera"* refers to *Aloe vera* of the Aloaceae family, the "moutan bark" refers to a root bark of *Paeonia moutan* of the Paeoniaceae family, the "laminaria" refers to an alga of the genus *Laminaria,* the "star fruit" refers to *Averrhoa carambola* of the Oxalidaceae family, the "cornflower" refers to *Centaurea cyanus* of the Asteraceae family, the "lemongrass" refers to *Cymbopogon citratus* of the Poaceae family, the "apricot kernel" refers to a seed of a plant belonging to the genus *Prunus* of the Rosaceae family, the "lemon" refers to *Citrus limon* of the Rutaceae family, the "sweet marjoram" refers to *Origanum majorana* of the Lamiaceae family, the "horse chestnut" refers to *Aesculus hippocastanum* of the Hippocastanaceae family, and the "rose hip" refers to a fruit of a plant belonging to the genus *Rosa* of the Rosaceae family, preferably *Rosa canina.* In addition the "rice" from which the "rice protein" and "rice oil" are derived refers to a seed of a plant belonging to the genus *Oryza* of the Poaceae family, and the "royal jelly" refers to a substance secreted from secretion glands (mandibular gland, hypopharyngeal gland) at the head of a worker bee.

In the case where the above-mentioned butcher's broom or the like is derived from a plant, the extract thereof may be an extract obtained from any part of the plant, for example, the whole plant, leaf, stem, germ, flower, bud, xylem, bark, thallus, root, rhizome, pseudobulb, corm, tuber, seed, fruit, sclerotium or resin, or a combination thereof. Specifically, preferred parts for preparing the extract are the rhizome for butcher's broom, the bark for Amur cork tree, the flower for *Hemerocallis fulva,* the flower or aerial part for rose balsam, the alga body for bladderwrack, the bark for birch, the leaf for *Aloe vera,* the root bark for moutan bark, the whole plant for laminaria, the leaf for star fruit, the flower for cornflower, the leaf for lemongrass, the seed for apricot kernel, the fruit for lemon, the leaf for sweet marjoram, the fruit for horse chestnut and the fruit for rose hip.

The parts listed above may be subjected to an extraction step directly, or may be subjected to the extraction step after pulverization, cutting, or drying.

The extract may be a commercially available one, or any solvent extracts obtained by an ordinary method, or diluted solutions thereof, concentrated solutions thereof, dried powders thereof, or pastes, or activated carbon-treated products thereof. The extracts can be obtained by extraction of parts to be extracted at room temperature or under heating, or by extraction using extraction tools such as a Soxhlet extractor.

The solvent to be used for extraction may be a polar solvent or a non-polar solvent. Specific examples of the solvent include: water; alcohols such as methanol, ethanol, propanol and butanol; polyhydric alcohols such as propylene glycol and butylene glycol; ketones such as acetone and methyl ethyl ketone; esters such as methyl acetate and ethyl acetate; linear and cyclic ethers such as tetrahydrofuran and diethyl ether; polyethers such as polyethylene glycol; hydrocarbons such as squalane, hexane, cyclohexane and petroleum ether; aromatic hydrocarbons such as toluene; halogenated hydrocarbons such as dichloromethane, chloroform and dichloroethane; supercritical carbon dioxide; pyridine; organic solvents such as other oils, e.g., an oil or fat and a wax; and mixtures thereof. It should be noted that one or more kinds selected from those solvents are preferably used. The solvent preferably includes water, an alcohol and an aqueous solution thereof, and the alcohol is preferably ethanol. More preferred solvents are water and an aqueous ethanol solution.

The blending ratio (volume ratio) of alcohol and water is preferably 0.001 to 100:99.999 to 0, more preferably 5 to 95:95 to 5, more preferably 20 to 80:80 to 20, more preferably 30 to 70:70 to 30, even more preferably 40 to 60:60 to 40. In the case of aqueous ethanol solution, the concentration of ethanol is preferably from 40 to 60 vol%.

The amount of the solvent used is preferably from 1 to 100 mL with respect to 1 g of the part (in terms of dry mass) to be extracted. Extraction conditions are not particularly limited as long as the extraction can be carried out satisfactorily. The extraction time is preferably from 3 minutes to 30 days, more preferably from 60 minutes to 14 days, and the extraction temperature is preferably from 0°C to the boiling point of the solvent, more preferably from 5 to 70°C. Usually, the extraction is carried out at a low temperature for a long period of time, or at high temperature for a short period of time.

Specific examples of extraction means for preparing the extract may include solid-liquid extraction, liquid-liquid extraction, soaking, decoction, leaching, reflux extraction, ultrasonic extraction, microwave extraction and stirring.

Examples of the extraction conditions include from 15 to 25°C for from 7 days to 14 days, 70°C for 5 hours and the like.

In addition, in the case where the extraction is performed in a shorter time, the solid-liquid extraction including stirring is desirably performed. Preferred examples of conditions of the solid-liquid extraction include stirring at from 40 to 100°C (preferably from 50 to 70°C) and from 100 to 1,000 rpm for from 30 to 300 minutes.

In order to prevent oxidation of the extract, there may further be used means for performing extraction under a so-called non-oxidative atmosphere while dissolved oxygen is removed by deaeration by boiling or passing of an inert gas such as nitrogen gas.

Of the extracts and the like, ginseng, hiba arborvitae, cucumber, birch and extracts thereof are preferred, and ginseng, hiba arborvitae and extracts thereof are more preferred, from the viewpoint of a blood circulation promoting effect and the viewpoint of an effect of improving skin circulation or skin metabolism.

The amount of the component (C) in the bath agent composition of the present invention is preferably from 2.0×10⁻⁵ to 0.6 mass%, more preferably from 2.0×10⁻⁴ to 0.3 mass%, even more preferably from 2.0×10⁻³ to 0.1 mass% in terms of dry weight, from the viewpoint of a blood circulation promoting effect and the viewpoint of an effect of improving skin circulation or skin metabolism.

It should be noted that the bath agent composition of the present invention may include a water insoluble excipient (D) from the viewpoint of more stably maintaining dispersibility of the component (C). With this, for example, the component (C) may be mixed with the water insoluble excipient (D) in advance to favorably disperse the component (C) in the composition, resulting in easily enhancing preservation stability. The component (D) is preferably porous powder from the viewpoint of favorably dispersing the component (C) in the composition, and for example, the oil absorption of the component (D) is preferably from 100 to 700 mL/100 g, more preferably from 200 to 600 mL/100 g.

Examples of the component (D) include crystalline silica, amorphous silica and amorphous aluminosilicate, and there may be preferably used, for example, a commercially available product such as FLORITE (manufactured by Tokuyama Corporation) (the name was changed into FLORITE R (Tomita Pharmaceutical Co. , Ltd.)), Sylopure (manufactured by Fuji Silysia Chemical Ltd.), Tokusil NR (manufactured by Tokuyama Corporation→manufactured by Oriental Silica Corporation), Tokusil NP (manufactured by Oriental Silica Corporation), TIXOLEX 25 (manufactured by Kofran Chemical Co. Ltd.), or Nipsil NA (manufactured by Tosoh Silica Corporation). Of those, crystalline silica such as FLORITE is preferred.

The amount of the component (D) in the bath agent composition of the present invention is preferably from 0.01 to 10 mass%, more preferably from 0.05 to 5 mass%, from the viewpoint of more stably maintaining dispersibility of the component (C).

The bath agent composition of the present invention may further include an oily component from the viewpoint of increasing a good warm feeling and skin care effect together with CGRP responsiveness provided by the component (C). Examples of the oily component include: a fatty acid ester such as cetyl octanoate, cetyl isooctanoate, butyl laurate, isoamyl laurate, methyl myristate, ethyl myristate, isopropyl myristate, octyldodecyl myristate, isopropyl palmitate, ethyl stearate, butyl stearate, isotridecyl isononanoate, isononyl isononanoate, isopropyl linoleate, isopropyl isostearate, isostearyl isostearate, methyl oleate or ethyl oleate; a glyceride such as glyceryl myristate or glyceryl 2-ethylhexanoate; a fatty acid glyceride such as glycerin tri(caprylate/caprate) or glyceryl tricaprylate; a fatty acid polyglyceride such as polyglyceryl-2 isostearate or polyglyceryl-2 triisostearate; a higher fatty acid such as lauric acid, myristic acid, palmitic acid, stearic acid, behenic acid, oleic acid, linoleic acid, linolenic acid, lanolin acid or isostearic acid; a higher alcohol such as lauryl alcohol, cetyl alcohol, stearyl alcohol, oleyl alcohol, cholesterol or 2-hexyldecanol; a hydrocarbon oil such as liquid paraffin, vaseline, paraffin, microcrystalline wax, ceresin, squalane, squalene, dioctylcyclohexane, or pristane; a natural oil or fat such as soybean oil, jojoba oil, avocado oil, almond oil, olive oil, cacao butter, sesame oil, persic oil, castor oil, coconut oil, mink oil, tallow or lard, or a hydrogenated oil obtained by hydrogenating the natural oil or fat; an essential oil such as mint oil, jasmine oil, camphor oil, bitter orange peel oil, rhyuno oil, turpentine oil, cinnamon oil, bergamot oil, orange oil, sweet flag oil, pineapple oil, lavender oil, clove oil, eucalyptus oil, thyme oil, peppermint oil, sage oil, menthol, cineol, eugenol, citral, citronellal, borneol, linalool, geraniol, camphor, thymol, spilanthol, pinene, limonene or terpene-based compound; and silicone oil. One kind may be used alone, or two or more kinds may be used in combination. Of those, one or more kinds selected from fatty acid esters are more preferred from the viewpoint of skin care effect.

The amount of the oily component in the bath agent composition of the present invention is preferably from 0.05 to 10 mass%, more preferably from 0.1 to 5 mass%, even more preferably from 0.2 to 3.5 mass%, from the viewpoint of suppressing an additional decrease in the water amount to provide soft skin even for skin having a decreased water amount in the horny layer to cause progressive hardening, the viewpoint of imparting warm feeling, and the viewpoint of maintaining good dispersibility to enhance preservation stability.

The bath agent composition of the present invention may appropriately include, as a component other than the above-mentioned components, a component that is usually used for a bath agent, for example, a non-ionic surfactant, a water soluble polymer, a saccharide, an inorganic salt other than carbonate, a vitamin, a protease, a bactericidal and antiseptic agent, a flavor, a dye or the like.

The bath agent composition of the present invention can be produced in accordance with a conventional method such as a compression granulation method such as extrusion granulation or a compression molding method using a compression tableting machine or a briquetting machine. The form of the composition may be any of powder, granule, particle, briquette tablet, tablet and the like. In addition, the composition may have a form obtained by granulating or molding part of the components in advance, mixing the resultant product with the other components, and molding the mixture. It should be noted that, when the component (C) is used, the component (C) is preferably mixed with the water insoluble excipient (D) in advance from the viewpoint of favorably dispersing the component (C).

The bath agent composition of the present invention is used as follows: the composition is dissolved in bath water to generate bubbles of carbon dioxide gas, and at least part of the body is soaked in the bath water. The body part to be soaked is not particularly limited, and the whole body may be soaked (whole-body bathing) or only a part of the body such as the neck, shoulder, hand, arm or leg may be soaked. Of those, the composition is preferably applied to so-called foot bathing for soaking the leg, in particular, the part below the knee or the part below the ankle in bath water, from the viewpoint of satisfactorily giving the effect of the present invention by continuously using the composition.

In order to further improve the blood circulation promoting effect and the vascular responsiveness improving effect to the heat load provided by the bath agent composition of the present invention, the bath agent composition of the present invention is capable of adjusting the pH of the bath water to preferably from 4 to 7, more preferably from 4.5 to 6.5.

In order to further improve the blood circulation promoting effect and the vascular responsiveness improving effect to the heat load provided by the bath agent composition of the present invention, the bath agent composition of the present invention is capable of adjusting the concentration of carbon dioxide gas in bath water to preferably from 30 to 1, 300 ppm, more preferably from 60 to 1,300 ppm, more preferably from 100 to 1,300 ppm, even more preferably from 800 to 1,300 ppm.

In addition, continuous use of the bath agent composition of the present invention can provide a more excellent blood circulation promoting effect and vascular responsiveness improving effect to heat load. Preferably, when the composition is used for bathing every day for continuous 4 days or more, a satisfactory effect is provided. The bathing is carried out for preferably 7 days or more, more preferably 10 days or more, even more preferably 14 days or more (continuous bathing). Such continuous bathing can satisfactorily exhibit the effect of the present invention, that is, the blood circulation promoting effect to provide good warm feeling and vascular responsiveness improving effect to heat load to improve skin circulation or skin metabolism and to suppress a decrease in the water amount of the horny layer, resulting in providing soft skin.

For example, as shown in FIG. 1(a), in the case of 4-week continuous bathing (foot bathing) using normal hot water (Comparative Example 6 to be described later), there is no significant difference between the initial stage and that after continuous bathing (FIG. 1(a)), while in the case of 4-week continuous bathing (foot bathing) using the bath agent composition of the present invention (bath agent composition of Example 1 to be described later), the blood flow can be significantly increased after continuous bathing as compared to that at the initial stage (FIG. 1(b)). In addition, as shown in FIG. 2, when the skin hardened due to a progressive dry state before bathing (foot bathing) (FIG. 2 (a)) is subjected to 4-week continuous bathing using the bath agent composition of the present invention (bath agent composition of Example 1 to be described later), the skin can be changed into soft skin with increased moisture (FIG. 2(b)).

In addition, in order to further improve the blood circulation promoting effect and vascular responsiveness improving effect to heat load provided by the bath agent composition of the present invention, the bathing temperature is preferably from 30 to 45°C, and the bathing time is preferably 3 minutes or more and 30 minutes or less, more preferably 5 minutes or more and 20 minutes or less, even more preferably 10 minutes or more and 15 minutes or less for each time of bathing.

The bath agent composition of the present invention is appropriately wrapped and preserved in a bag-like wrapping. In this case, the water vapor permeability of the material of the bag-like wrapping to be used in the present invention is 0.6 g/m²·day or less, preferably 0.3 g/m²·day or less under conditions of 40 °C and 90%RH, and even more preferably, the bag-like wrapping does not substantially transmit water vapor. An example of the material of the bag-like wrapping having such water vapor permeability is a laminate film made of aluminum having a thickness of from 5 to 10 µm, and a preferred example of the material of the bag-like wrapping is a transparent material such as a polyethylene terephthalate (PET) film subjected to glass vapor deposition treatment.

With regard to the embodiments of the present invention described above, the following bath agent composition is further disclosed.
[1] A bath agent composition, comprising the following components (A) to (C):
   (A) a carbonate comprising 30 mass% or more of a dialkali metal carbonate (A1);
   (B) an organic acid; and
   (C) a CGRP response promoter.
[2] The bath agent composition according to Item [1], in which the amount of the component (A) is preferably from 20 to 70 mass%, more preferably from 25 to 65 mass%, even more preferably from 30 to 60 mass%.
[3] The bath agent composition according to Item [1] or [2], further comprising a carbonate as a carbonate (A2), other than the component (A1) in the component (A), in which the component (A2) comprises preferably a monoalkali metal carbonate, more preferably sodium hydrogen carbonate or potassium hydrogen carbonate.
[4] The bath agent composition according to Items [1] to [3], in which the amount of the monoalkali metal carbonate in the component (A) is preferably 60 mass% or less, more preferably 50 mass% or less, even more preferably from 0 to 40 mass%.
[5] The bath agent composition according to Items [1] to [4], in which the component (B) is preferably an organic acid that is solid at room temperature (25°C), more preferably one or more kinds selected from the group consisting of malic acid, tartaric acid, citric acid, maleic acid, succinic acid, phthalic acid, fumaric acid, glutaric acid, adipic acid, benzoic acid, salicylic acid and oxalic acid, more preferably one or more kinds selected from the group consisting of citric acid, fumaric acid, malic acid, tartaric acid, succinic acid and oxalic acid, even more preferably citric acid or fumaric acid.
[6] The bath agent composition according to Items [1] to [5], in which the amount of the component (B) is preferably from 20 to 70 mass%, more preferably from 25 to 65 mass%, even more preferably from 30 to 60 mass%.
[7] The bath agent composition according to Items [1] to [6], in which the amount of the component (C) is preferably from 2.0×10⁻⁵ to 0.6 mass%, more preferably from 2.0×10⁻⁴ to 0.3 mass%, even more preferably from 2.0×10⁻³ to 0.1 mass% in terms of dry weight.
[8] The bath agent composition according to Items [1] to [7], in which the component (C) is preferably one or more kinds selected from the group consisting of ginseng, hiba arborvitae, cucumber butcher's broom, Amur cork tree, *Hemerocallis fulva,* rose balsam, bladderwrack, birch, rice protein, rice oil, *Aloe vera,* moutan bark, laminaria, star fruit, cornflower, lemongrass, apricot kernel, lemon, sweet marjoram, horse chestnut, royal jelly, rose hip and extracts thereof, more preferably one or more kinds selected from the group consisting of ginseng, hiba arborvitae, cucumber, birch and extracts thereof.
[9] The bath agent composition according to Items [1] to [8], further comprising an insoluble excipient (D).
[10] The bath agent composition according to Items [1] to [9], in which the component (D) has an oil absorption of preferably from 100 to 700 mL/100 g, more preferably from 200 to 600 mL/100 g.
[11] The bath agent composition according to Items [1] to [10], in which the component (D) is preferably crystalline silica, amorphous silica or amorphous aluminosilicate.
[12] The bath agent composition according to Items [9] to [11], in which the amount of the component (D) is preferably from 0.01 to 10 mass%, more preferably from 0.05 to 5 mass%.
[13] The bath agent composition according to Items [9] to [12], in which the composition is obtained by mixing the component (C) and the component (D), in advance.
[14] The bath agent composition according to Items [1] to [13], further comprising preferably from 0. 05 to 10 mass%, more preferably from 0.1 to 5 mass% of an oily component.
[15] The bath agent composition according to Items [1] to [14], in which the composition is capable of adjusting the pH of bath water to preferably from 4 to 7, more preferably from 4.5 to 6.5.
[16] The bath agent composition according to Items [1] to [15], in which the composition is capable of adjusting the concentration of carbon dioxide gas in bath water to preferably from 30 to 1,300 ppm, more preferably from 60 to 1,300 ppm.
[17] The bath agent composition according to Items [1] to [16], in which the composition is preferably continuously used, more preferably continuously used for 4 days or more, more preferably continuously used for 7 days or more, even more preferably continuously used for 10 days or more.
[18] The bath agent composition according to Items [1] to [17], in which the composition is preferably used for foot bathing.

### Examples

Hereinafter, the present invention is specifically described by way of Examples. It should be noted that the amount of each component is represented in a mass% unit unless otherwise specifically stated in tables.

### [Production Example 1: Preparation of ginseng extract]

To 100 g of the root of *Panax ginseng* (source from China), 1,000 mL of an aqueous solution of 50% ethanol was added, and extraction was carried out by soaking at room temperature (25°C) for 7 days, followed by filtration, to thereby obtain 840 mL of a ginseng extract (evaporation residue: 2.8 w/v%, determined by Japanese Standards of Quasi-drug Ingredients (10 mL, 105°C, 6 hours))

### [Production Example 2: Preparation of hiba arborvitae extract]

To 100 g of the leaf and small branch of hiba arborvitae *(Thujopsis dolabrate*) (source from Japan), 1,000 mL of ethanol was added, and extraction was carried out by soaking at room temperature (25°C) for 7 days, followed by filtration, to thereby obtain 900 mL of a hiba arborvitae extract (evaporation residue: 0.8 w/v%, determined by Japanese Standards of Quasi-drug Ingredients (10 mL, 105°C, 6 hours))

### [Production Example 3: Preparation of cucumber extract]

To 100 g of the fruit of cucumber *(Cucumis sativus*) (source from Japan), 1,000 mL of an aqueous solution of 50% ethanol was added, and extraction was carried out by soaking at room temperature (25°C) for 7 days, followed by filtration, to thereby obtain 1000 mL of a cucumber extract (evaporation residue: 0.2w/v%, determined by Japanese Standards of Quasi-drug Ingredients (10 mL, 105°C, 6 hours))

[Production Example 4: Preparation of birch extract] To 100 g of the bark and xylem of Japanese white birch *(Betula platyphylla*) (source from Japan), 1,000 mL of an aqueous solution of 50% ethanol was added, and extraction was carried out by soaking at room temperature (25°C) for 7 days, followed by filtration, to thereby obtain 1,000 mL of a birch extract (evaporation residue: 0.1 w/v%, determined by Japanese Standards of Quasi-drug Ingredients (10 mL, 105°C, 6 hours))

### [Examples 1 to 3 and Comparative Example 1]

Bath agents were prepared by mixing the ingredient according to the formulae shown in Table 1, using the ginseng extract obtained in Production Example 1. It should be noted that the ginseng extract was mixed with sodium silicate (FLORITE) in advance, and the resultant was mixed with the other components to prepare the bath agents.

The resultant bath agents were subjected to the following tests. Table 1 shows the results.

### [Test Example 1: Preservation test]

Each bath agent was wrapped with an aluminum (7 µm) laminate film and preserved for 6 months at a temperature of 40°C and a relative humidity of 75%RH, and then expandability of the wrapping pack was visually evaluated.

AA: No expansion was observed in the wrapping pack at all.

A: No expansion was observed in the wrapping pack, or only a little expansion was observed in the wrapping pack, and there was still an enough space left to swell in the wrapping pack.

C: Wrapping pack bursted or clearly expanded, and there was no space left to swell in the wrapping pack.

**[Table 1]**

| Component | | Example 1 | Example 2 | Example 3 | Comparative Example 1 |
|---|---|---|---|---|---|
| (A) Carbonate | (A1) Sodium carbonate | 31.3 | 31.3 | 16.0 | 5.0 |
| | (A2) Sodium hydrogen carbonate | - | - | 15.3 | 26.0 |
| (B) Organic acid | Citric acid | 58.8 | 58.8 | 58.8 | 59.1 |
| (C) CGRP response promoter | Ginseng extract | 5.0 | 5.0 | 5.0 | 5.0 |
| (D) Water insoluble excipient | FLORITE^{*1} | 2.5 | - | 2.5 | 2.5 |
| | Tokusil NR^{*2} | - | 2.5 | - | - |
| Isopropyl palmitate | | 0.21 | 0.21 | 0.21 | 0.21 |
| Octyl dodecyl myristate | | 0.06 | 0.06 | 0.06 | 0.06 |
| Polyoxyethylene sorbitol tetraoleate | | 0.09 | 0.09 | 0.09 | 0.09 |
| Polyoxyethylene stearyl ether | | 0.04 | 0.04 | 0.04 | 0.04 |
| Magnesium oxide | | 2.0 | 2.0 | 2.0 | 2.0 |
| Total | | 100.0 | 100.0 | 100.0 | 100.0 |
| Amount (mass%) of dialkali carbonate in component (A) | | 100.0 | 100.0 | 51.1 | 16.1 |
| Amount (mass%) of (C) CGRP response promoter in terms of dry weight | | 0.14 | 0.14 | 0.14 | 0.14 |
| Amount (g) per use | | 45.0 | 45.0 | 45.0 | 45.0 |
| Evaluation | | | | | |
| Preservation test | | AA | AA | A | C |

| | | | | | |
|---|---|---|---|---|---|
| *1: manufactured by Tokuyama Corporation, equivalent of FLORITE R (manufactured by Tomita Pharmaceutical Co., Ltd.), oil absorption: from 400 to 600 mL/100 g *2: manufactured by Oriental Silicas Corporation, oil absorption: 250 mL/100 g | | | | | |

### [Examples 4 and 5 and Comparative Examples 2 to 5]

Bath agents were prepared by mixing the ingredients according to the formulae shown in Table 2, using the ginseng extract obtained in Production Example 1. It should be noted that, in Comparative Example 5, the following test was carried out in normal hot water without using any bath agent. In addition, the ginseng extract was mixed with calcium silicate (FLORITE) in advance, and then mixed with the other components to prepare bath agents.

The resultant bath agents were subjected to the following tests. Table 2 shows the results including the result of Example 1.

### [Test Example 2]

Each bath agent was put in 6 L of 40°C hot water in the amount shown in Table 2, to prepare hot bath water having a carbon dioxide gas concentration of from 800 to 1,300 ppm and a pH of from 4.5 to 6.5, and five test subjects soaked their feet below their ankles for 15 minutes for foot bathing. Then, cutaneous blood flows (mL/min) were measured before and immediately after the foot bathing. The blood flow of each subject before the start of the foot bathing was defined as 100, the blood flow of each subject 15 minutes after the start of the foot bathing was represented as an index, and then the results of the five subjects were averaged. It should be noted that the cutaneous blood flow was measured at the dorsum of the foot by attaching an electrode using a laser Doppler blood-flowmeter (ALF21, manufactured by ADVANCE Co., Ltd.).

### [Test Example 3]

Each bath agent was put in 6 L of 40°C hot water in the amount shown in Table 2 to prepare hot bath water with carbon dioxide gas concentration of from 800 to 1, 300 ppm and a pH of from 4.5 to 6.5, and five test subjects soaked their feet below their ankles for 10 minutes once a day for 4-week continuous foot bathing. Evaluation was carried out on the vascular responsiveness improving effect to heat load and cutaneous symptom ameliorating effect according to the following methods and criteria.

### «Evaluation of vascular responsiveness improving effect to heat load»

The heat load on the skin was carried out by controlling the temperature of a heat load probe which embeds a Peltier device using a hot and cold stimulating device (manufactured by Physio-Tech Co. , Ltd.). The probe for measuring the blood flow was fixed at the center of the heat load probe, and cutaneous blood flow was measured using a laser Doppler blood-flowmeter (ALF21, manufactured by ADVANCE Co., Ltd.). Specifically, first, the initial skin temperature of each of the five test subjects at rest was adjusted to 30°C and acclimated well, and then a heat was loaded using the hot and cold stimulating device to raise the skin temperature to 42°C. The temperature was kept at 42°C for 5 minutes and then returned to 30°C again. The cutaneous blood flow under the heat-stress was measured using the device for measurement of the cutaneous blood flow (laser Doppler blood-flowmeter). The blood flow before the continuous foot bathing was defined as 100, and evaluation was carried out according to the following criteria to calculate an average value of the results of the five test subjects.
A: Blood flow after continuous foot bathing was 115 or more.
B: Blood flow after continuous foot bathing was 105 or more and less than 115.
C: Blood flow after continuous foot bathing was less than 105.

### «Evaluation of cutaneous symptom ameliorating effect»

Five expert panelists visually observed changes in the horny layers of the heels of the five test subjects between before and after the continuous foot bathing, and carried out evaluation according to the following criteria.

Score 3: Amelioration of dry skin and improvement of softness were clearly observed as compared to before the continuous foot bathing.

Score 2: Amelioration of dry skin and improvement of softness were observed as compared to before the continuous foot bathing.

Score 1: Amelioration of dry skin and improvement of softness were hardly observed as compared to before the continuous foot bathing.

Score 0: Amelioration of dry skin and improvement of softness were not observed as compared to before the continuous foot bathing.

An average of the scores for the respective test subjects was calculated, and evaluation was carried out according to the following criteria.

AA: Average was 2.5 or more.
A: Average was 1.5 or more and less than 2.5.
B: Average was 0.5 or more and less than 1.5.
C: Average was less than 0.5.

**[Table 2]**

| Component | | Example 1 | Example 4 | Example 5 | Comparative Example 2 | Comparative Example 3 | Comparative Example 4 | Comparative Example 5 |
|---|---|---|---|---|---|---|---|---|
| (A) Carbonate | (A1) Sodium carbonate | 31.3 | 31.4 | 10.0 | 34.0 | 33.6 | - | - |
| | (A2) Sodium hydrogen carbonate | - | - | 21.4 | - | - | - | - |
| (B) Organic acid | Citric acid | 58.8 | 59.1 | 59.1 | 64.0 | 64.0 | - | - |
| (C) CGRP response promoter | Ginseng extract | 5.0 | 5.0 | 5.0 | - | - | 100.0 | - |
| (D) Water insoluble excipient | FLORITE^{*1} | 2.5 | 2.5 | 2.5 | - | - | - | - |
| Isopropyl palmitate | | 0.21 | - | - | - | 0.21 | - | - |
| Octyl dodecyl myristate | | 0.06 | - | - | - | 0.06 | - | - |
| Polyoxyethylene sorbitol tetraoleate | | 0.09 | - | - | - | 0.09 | - | - |
| Polyoxyethylene stearyl ether | | 0.04 | - | - | - | 0.04 | - | - |
| Magnesium oxide | | 2.0 | 2.0 | 2.0 | 2.0 | 2.0 | - | - |
| Total | | 100.0 | 100.0 | 100.0 | 100.0 | 100.0 | 100.0 | - |
| Amount (mass%) of dialkali carbonate in component (A) | | 100.0 | 100.0 | 31.8 | 100.0 | 100.0 | - | - |
| Amount (mass%) of (C) CGRP response promoter in terms of dry weight | | 0.14 | 0.14 | 0.14 | - | - | 2.80 | - |
| Amount (g) per use | | 45.0 | 45.0 | 45.0 | 45.0 | 45.0 | 2.5 | - |
| Evaluation | | | | | | | | |
| Cutaneous blood flow (represented as index relative to that before foot bathing defined as 100) | | 400 | 400 | 400 | 240 | 240 | 200 | 190 |
| Vascular responsiveness improving effect | | A | A | A | B | B | B | C |
| Cutaneous symptom ameliorating effect | | AA | A | A | B | B | C | C |

| | | | | | | | | |
|---|---|---|---|---|---|---|---|---|
| *1: The same as that in Table 1. | | | | | | | | |

The results of Tables 1 and 2 reveal that the bath agents of Examples have excellent preservation stability, and usage of the bath agents for bathing can increase the cutaneous blood flow immediately after foot bathing. Further, the results reveal that the continuous bathing can significantly improve the vascular responsiveness to heat load and can ameliorate the symptom of the skin.

### [Examples 6 to 9]

Bath agents were prepared according to the formulae shown in Table 3 using the hiba arborvitae extract and cucumber extract obtained in Production Examples 2 and 3. Usage of either one of the bath agents, the vascular responsiveness improving effect to heat load was observed, and the dry skin of the heel was significantly ameliorated and was changed to soft skin.

**[Table 3]**

| Component | | Example 6 | Example 7 | Example 8 | Example 9 |
|---|---|---|---|---|---|
| (A) Carbonate | (A1) Sodium carbonate | 31.30 | 31.40 | 31.30 | 31.40 |
| | (A2) Sodium hydrogen carbonate | - | - | - | - |
| (B) Organic acid | Citric acid | 58.80 | 59.10 | 58.80 | 59.10 |
| (C) CGRP response promoter | Hiba arborvitae extract | 5.00 | 5.00 | - | - |
| | Cucumber extract | - | - | 5.00 | 5.00 |
| (D) Water insoluble excipient | FLORITE^{*1} | 2.50 | 2.50 | 2.50 | 2.50 |
| Isopropyl palmitate | | 0.21 | - | 0.21 | - |
| Octyl dodecyl myristate | | 0.06 | - | 0.06 | - |
| Polyoxyethylene sorbitol tetraoleate | | 0.09 | - | 0.09 | - |
| Polyoxyethylene stearyl ether | | 0.04 | - | 0.04 | - |
| Magnesium oxide | | 2.00 | 2.00 | 2.00 | 2.00 |
| Total | | 100.00 | 100.00 | 100.00 | 100.00 |
| Amount (mass%) of dialkali carbonate in component (A) | | 100 | 100 | 100 | 100 |
| Amount (mass%) of (C) CGRP response promoter in terms of dry weight | | 4.00E-03 | 4.00E-03 | 1.00E-03 | 1.00E-03 |
| Amount (g) per use | | 45.00 | 45.00 | 45.00 | 45.00 |

| | | | | | |
|---|---|---|---|---|---|
| *1: The same as that in Table 1. | | | | | |

### [Examples 10 to 15]

Bath agents were prepared according to the formulae shown in Table 4 using the ginseng extract obtained in Production Example 1 and using different kinds or different amount of the organic acids as the component (B).

The resultant bath agents were used to evaluate the vascular responsiveness improving effect and the cutaneous symptom ameliorating effect in the same manner as above and to evaluate a joint flexibilizing effect according to the following method and criteria.

The results including the results of Example 1 are shown in table 4.

### «Evaluation of joint flexibilizing effect»

Plantarflexion and dorsiflexion angles of the ankle joints were measured in a supine position. The position at which the sole of the foot is vertical to the floor was defined as 0°. The angle of the maximum plantarflexion provided by the subject himself or herself was defined as plantarflexion angle, while the angle of the maximum dorsiflexion provided by the subject himself or herself was defined as dorsiflexion angle. The sum of the angles was defined as a value (°) representing a range of plantarflexion-dorsiflexion movement of the ankle joint. An increase (°) in the value after foot bathing compared to the value before foot bathing was determined and was used as an evaluation index for the joint flexibilizing effect.

**[Table 4]**

| Component | | Example 1 | Example 10 | Example 11 | Example 12 | Example 13 | Example 14 | Example 15 |
|---|---|---|---|---|---|---|---|---|
| (A) Carbonate | (A1) Sodium carbonate | 31.30 | 12.00 | 31.30 | 31.30 | 31.30 | 31.30 | 31.30 |
| | (A2) Sodium hydrogen carbonate | 0.00 | 23.00 | - | - | - | - | - |
| (B) Organic acid | Citric acid | 58.80 | 50.00 | - | - | - | - | 30.00 |
| | Fumaric acid | - | - | 58.80 | - | - | - | - |
| | Malic acid | - | - | - | 58.80 | - | - | 28.80 |
| | Tartaric acid | - | - | - | - | 58.80 | - | - |
| | Succinic acid | - | - | - | - | - | 30.00 | - |
| | Oxalic acid | - | - | - | - | - | 28.80 | - |
| (C) CGRP response promoter | Ginseng extract | 5.00 | 5.00 | 5.00 | 5.00 | 5.00 | 5.00 | 5.00 |
| (D) Water insoluble excipient | FLORITE*1 | 2.50 | 4.00 | 2.50 | 2.50 | 2.50 | 2.50 | 2.50 |
| Isopropyl palmitate | | 0.21 | 0.53 | 0.21 | 0.21 | 0.21 | 0.21 | 0.21 |
| Octyl dodecyl myristate | | 0.06 | 0.15 | 0.06 | 0.06 | 0.06 | 0.06 | 0.06 |
| Polyoxyethylene sorbitol tetraoleate | | 0.09 | 0.23 | 0.09 | 0.09 | 0.09 | 0.09 | 0.09 |
| Polyoxyethylene stearyl ether | | 0.04 | 0.10 | 0.04 | 0.04 | 0.04 | 0.04 | 0.04 |
| Magnesium oxide | | 2.00 | 5.00 | 2.00 | 2.00 | 2.00 | 2.00 | 2.00 |
| Total | | 100.0 | 100.0 | 100.0 | 100.0 | 100.0 | 100.0 | 100.0 |
| Amount (mass%) of dialkali carbonate in component (A) | | 100.0 | 34.3 | 100.0 | 100.0 | 100.0 | 100.0 | 100.0 |
| Amount (mass%) of (C) CGRP response promoter in terms of dry weight | | 0.14 | 0.14 | 0.14 | 0.14 | 0.14 | 0.14 | 0.14 |
| Amount (g) per use | | 45.00 | 45.00 | 45.00 | 45.00 | 45.00 | 45.00 | 45.00 |
| Evaluation | | | | | | | | |
| Vascular responsiveness improving effect | | A | A | A | A | A | A | A |
| Cutaneous symptom ameliorating effect | | AA | AA | AA | AA | AA | AA | AA |
| Joint flexibilizing effect (°) | | 7.5 | 7.5 | 7.5 | 7.5 | 7.5 | 7.5 | 7.5 |

| | | | | | | | | |
|---|---|---|---|---|---|---|---|---|
| *1: The same as that in Table 1. | | | | | | | | |

### [Examples 16 to 21]

Bath agents were prepared according to the formulae shown in Table 5 using the ginseng extract obtained in Production Example 1 and using different amount of the carbonate as the component (A) and different amount of the dialkali carbonate in the component (A).

The resultant bath agents were used to evaluate the vascular responsiveness improving effect, the cutaneous symptom ameliorating effect and the joint flexibilizing effect in the same manner as above.

The results are shown in table 5.

**[Table 5]**

| Component | | Example 16 | Example 17 | Example 18 | Example 19 | Example 20 | Example 21 |
|---|---|---|---|---|---|---|---|
| (A) Carbonate | (A1) Sodium carbonate | 22.00 | 12.00 | 15.00 | 14.00 | 8.50 | 22.50 |
| | (A2) Sodium hydrogen carbonate | 11.00 | 28.00 | 34.00 | 7.00 | 18.00 | 12.00 |
| (B) Organic acid | Fumaric acid | 60.50 | 53.50 | 44.50 | 48.50 | 42.00 | 34.00 |
| (C) CGRP response promoter | Ginseng extract | 1.00 | 1.00 | 1.00 | 0.10 | 0.10 | 0.10 |
| (D) Water insoluble excipient | FLORITE^{*1} | 3.00 | 3.00 | 3.00 | 6.00 | 6.00 | 6.00 |
| Glucose | | - | - | - | 20.40 | 20.40 | 20.40 |
| Isopropyl palmitate | | 0.27 | 0.27 | 0.27 | 0.53 | 0.53 | 0.53 |
| Octyl dodecyl myristate | | 0.08 | 0.08 | 0.08 | 0.15 | 0.15 | 0.15 |
| Polyoxyethylene sorbitol tetraoleate | | 0.12 | 0.12 | 0.12 | 0.23 | 0.23 | 0.23 |
| Polyoxyethylene stearyl ether | | 0.05 | 0.05 | 0.05 | 0.10 | 0.10 | 0.10 |
| Magnesium oxide | | 2.00 | 2.00 | 2.00 | 4.00 | 4.00 | 4.00 |
| Total | | 100.0 | 100.0 | 100.0 | 101.0 | 100.0 | 100.0 |
| Amount (mass%) of dialkali carbonate in component (A) | | 0.67 | 0.30 | 0.31 | 0.67 | 0.32 | 0.65 |
| Amount (mass%) of (C) CGRP response promoter in terms of dry weight | | 2.80E-02 | 2.80E-02 | 2.80E-02 | 2.80E-03 | 2.80E-03 | 2.80E-03 |
| Amount (g) per use | | 45.00 | 45.00 | 45.00 | 45.00 | 45.00 | 45.00 |
| Evaluation | | | | | | | |
| Vascular responsiveness improving effect | | A | A | A | A | A | A |
| Cutaneous symptom ameliorating effect | | AA | AA | AA | AA | AA | AA |
| Joint flexibilizing effect (°) | | 7.5 | 7.5 | 7.5 | 7.5 | 7.5 | 7.5 |

| | | | | | | | |
|---|---|---|---|---|---|---|---|
| *1: The same as that in Table 1. | | | | | | | |

### [Examples 22 to 30]

Bath agents were prepared according to the formulae shown in Table 6 using the ginseng extract, hiba arborvitae extract, cucumber extract and birch extract obtained in Production Examples 1 to 4.

The resultant bath agents were used to evaluate the vascular responsiveness improving effect, the cutaneous symptom ameliorating effect and the joint flexibilizing effect in the same manner as above.

The results are shown in table 6.

**[Table 6]**

| Component | | Example 22 | Example 23 | Example 24 | Example 25 | Example 26 | Example 27 | Example 28 | Example 29 | Example 30 |
|---|---|---|---|---|---|---|---|---|---|---|
| (A) Carbonate | (A1) Sodium carbonate | 31.30 | 31.30 | 31.30 | 31.30 | 25.00 | 25.00 | 25.00 | 25.00 | 25.00 |
| | (A2) Sodium hydrogen carbonate | 0.00 | 0.00 | 0.00 | 0.00 | 8.00 | 8.00 | 8.00 | 8.00 | 8.00 |
| (B) Organic acid | Citric acid | - | - | - | - | 58.00 | 58.00 | 58.00 | 58.00 | 58.00 |
| | Fumaric acid | 58.80 | 58.80 | 58.80 | 58.80 | - | - | - | - | - |
| (C) CGRP response promoter | Ginseng extract | 5.00 | - | - | - | 1.00 | 0.50 | 0.10 | 0.05 | 0.008 |
| | Hiba arborvitae extract | - | 5.00 | - | - | - | - | - | - | - |
| | Cucumber extract | - | - | 5.00 | - | - | - | - | - | - |
| | Birch extract | - | - | - | 5.00 | - | - | - | - | - |
| (D) Water insoluble excipient | FLORITE^{*1} | 2.50 | 2.50 | 2.50 | 2.50 | 5.00 | 5.50 | 5.90 | 5.95 | 5.992 |
| Oily component | | 0.40 | 0.40 | 0.40 | 0.40 | 1.00 | 1.00 | 1.00 | 1.00 | 1.00 |
| Isopropyl palmitate | | 0.21 | 0.21 | 0.21 | 0.21 | 0.53 | 0.53 | 0.53 | 0.53 | 0.53 |
| Octyl dodecyl myristate | | 0.06 | 0.06 | 0.06 | 0.06 | 0.15 | 0.15 | 0.15 | 0.15 | 0.15 |
| Polyoxyethylene sorbitol tetraoleate | | 0.09 | 0.09 | 0.09 | 0.09 | 0.23 | 0.23 | 0.23 | 0.23 | 0.23 |
| Polyoxyethylene stearyl ether | | 0.04 | 0.04 | 0.09 | 0.09 | 0.10 | 0.10 | 0.10 | 0.10 | 0.10 |
| Magnesium oxide | | 2.00 | 2.00 | 2.00 | 2.00 | 2.00 | 2.00 | 2.00 | 2.00 | 2.00 |
| Total | | 100.40 | 100.40 | 100.40 | 100.40 | 101.00 | 101.00 | 101.00 | 101.00 | 101.000 |
| Amount (mass%) of dialkali carbonate in component (A) | | 100.00 | 100.00 | 100.00 | 100.00 | 0.76 | 0.76 | 0.76 | 0.76 | 0.76 |
| Amount (mass%) of (C) CGRP response promoter in terms of dry weight | | 0.14 | 4.00E-03 | 1.00E-03 | 6.50E-04 | 2.80E-02 | 1.40E-02 | 2.80E-03 | 1.40E-03 | 2.24E-04 |
| Amount (g) per use | | 45.00 | 45.00 | 45.00 | 45.00 | 45.00 | 45.00 | 45.00 | 45.00 | 45.00 |
| Evaluation | | | | | | | | | | |
| Vascular responsiveness improving effect | | A | A | A | B | A | B | B | B | B |
| Cutaneous symptom ameliorating effect | | AA | AA | AA | A | AA | A | A | A | A |
| Joint flexibilizing effect (°) | | 7.3 | 7.3 | 7.1 | 6.8 | 7.5 | 7.1 | 6.8 | 6.1 | 5.5 |

| | | | | | | | | | | |
|---|---|---|---|---|---|---|---|---|---|---|
| *1: The same as that in Table 1. | | | | | | | | | | |

### [Examples 31 to 34]

Bath agents were prepared according to the formulae shown in Table 7 using the ginseng extract obtained in Production Example 1.

The resultant bath agents were used to evaluate the vascular responsiveness improving effect, the cutaneous symptom ameliorating effect and the joint flexibilizing effect in the same manner as above.

Table 7 shows the results.

**[Table 7]**

| Component | | Example 31 | Example 32 | Example 33 | Example 34 |
|---|---|---|---|---|---|
| (A) Carbonate | (A1) Sodium carbonate | 10.00 | 10.00 | 10.00 | 10.00 |
| | (A2) Sodium hydrogen carbonate | 22.00 | 22.00 | 22.00 | 22.00 |
| (B) Organic acid | Tartaric acid | 50.00 | 50.00 | 50.00 | 50.00 |
| (C) CGRP response promoter | Ginseng extract | 1.00 | 1.00 | 1.00 | 1.00 |
| (D) Water insoluble excipient | FLORITE*1 | 14.70 | 14.00 | 12.00 | 10.00 |
| Isopropyl palmitate | | 0.16 | 0.53 | 1.59 | 2.65 |
| Octyl dodecyl myristate | | 0.05 | 0.15 | 0.45 | 0.75 |
| Polyoxyethylene sorbitol tetraoleate | | 0.07 | 0.23 | 0.69 | 1.15 |
| Polyoxyethylene stearyl ether | | 0.03 | 0.10 | 0.30 | 0.50 |
| Magnesium oxide | | 2.00 | 2.00 | 2.00 | 2.00 |
| Total | | 100.0 | 100.0 | 100.0 | 100.1 |
| Amount (mass%) of dialkali carbonate in component (A) | | 0.31 | 0.31 | 0.31 | 0.31 |
| Amount (mass%) of (C) CGRP response promoter in terms of dry weight | | 0.14 | 0.14 | 2.80E-02 | 1.40E-01 |
| Amount (g) per use | | 45.00 | 45.00 | 45.00 | 45.00 |
| Evaluation | | | | | |
| Vascular responsiveness improving effect | | A | A | A | A |
| Cutaneous symptom ameliorating effect | | AA | AA | AA | AA |
| Joint flexibilizing effect (°) | | 7.5 | 7.5 | 7.5 | 7.5 |

| | | | | | |
|---|---|---|---|---|---|
| *1: The same as that in Table 1. | | | | | |

The results of Tables 4 to 7 reveal that, in the case of using any one of the bath agents, the continuous bathing can significantly improve the vascular responsiveness to heat load, can effectively ameliorate the skin symptom and can provide an effect of flexibilizing the joint.

## Claims

1. A bath agent composition, comprising the following components (A) to (C):
(A) a carbonate comprising 30 mass% or more of a dialkali metal carbonate (A1);
(B) an organic acid; and
(C) a CGRP response promoter.

2. The bath agent composition according to claim 1, wherein an amount of the component (A) is from 20 to 70 mass%.

3. The bath agent composition according to claim 1 or 2, further comprising a carbonate as a carbonate (A2), other than the component (A1) in the component (A), wherein the component (A2) comprises a monoalkali metal carbonate.

4. The bath agent composition according to claim 3, wherein an amount of the monoalkali metal carbonate is 60 mass% or less in the component (A).

5. The bath agent composition according to claim 4, wherein the monoalkali metal carbonate is sodium hydrogen carbonate or potassium hydrogen carbonate.

6. The bath agent composition according to any one of claims 1 to 5, wherein an amount of the component (B) is from 20 to 70 mass%.

7. The bath agent composition according to any one of claims 1 to 6, wherein an amount of the component (C) is from 2.0×10⁻⁵ to 0.6 mass% in terms of dry weight.

8. The bath agent composition according to any one of claims 1 to 7, wherein the component (C) is one or more kinds selected from the group consisting of ginseng, hiba arborvitae, cucumber, birch and extracts thereof.

9. The bath agent composition according to any one of claims 1 to 8, wherein the composition is continuously used.
